(19) Europäisches Patentamt
European Patent Office
Office européen des brevets

(11) Numéro de publication: **0 352 150**
**A1**

(12) **DEMANDE DE BREVET EUROPEEN**

(21) Numéro de dépôt: **89401698.9**

(22) Date de dépôt: **16.06.89**

(51) Int. Cl.5: **C 07 B 37/04**
C 07 B 39/00, C 07 B 45/04,
C 07 C 25/02, C 07 C 49/76,
C 07 C 309/39,
C 07 C 311/16, C 07 F 7/08,
C 07 C 17/22, C 07 C 45/00

(30) Priorité: **27.06.88 FR 8808581**

(43) Date de publication de la demande:
**24.01.90 Bulletin 90/04**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Benneteau, Bernard**
**3, rue Paul Verlaine**
**F-33740 Ares (FR)**

**Dunogues, Jacques**
**22, avenue du Président Poincaré**
**F-33400 Talence (FR)**

**Krempp, Michèle**
**8, rue Kertzfeld**
**F-67230 Benfeld (FR)**

(74) Mandataire: **Vignally, Noel et al**
**Rhône-Poulenc Chimie Service Brevets Chimie 25 Quai Paul Doumer**
**F-92408 Courbevoie Cédex (FR)**

(54) **Procédé de préparation de dérivés métasubstitués du fluoro- ou des alkylbenzènes.**

(57) La présente invention concerne un procédé de préparation de dérivés métasubstitués du fluoro- ou des alkylbenzènes à partir de fluoro- ou d'alkylbenzènes par passage par un dérivé disilicié, monodésilylation en ortho du dérivé disilicié, et fonctionnalisation en méta du dérivé monosilicié.

Elle permet d'atteindre des dérivés métasubstitués à partir de composés de départ ortho ou paradirecteurs.

EP 0 352 150 A1

Bundesdruckerei Berlin

## Description

### PROCEDE DE PREPARATION DÈ DERIVES METASUBSTITUES DU FLUORO- OU DES ALKYLBENZENES

La présente invention concerne un nouveau procédé de substitution en méta de dérivés benzéniques monosubstitués par un groupe ortho-/para-directeur. Elle concerne plus particulièrement la préparation de fluoro- et d'alkylbenzènes substitués en méta par un groupe acyle, halogéno-, sulfonyle.

Il est connu depuis longtemps que les dérivés du type fluorobenzène ou alkylbenzènes, lorsqu'ils sont soumis à une réaction de substitution électrophile, donnent exclusivement des dérivés ortho- ou para-substitués. Or certaines synthèses chimiques exigent la présence de dérivés par exemple du fluorobenzène méta-substitué. Les voies classiques de la chimie ne permettent pas d'arriver à de tels dérivés de manière simple. La chimie est donc toujours à la recherche d'une méthode facile de synthèse de dérivés substitués en méta du fluorobenzène ou d'alkylbenzènes.

Il était décrit d'après l'article publié par Eaborn dans Organometallics in Chemicals Synthesis, 1, (1970), 151 que la substitution en méta d'un méthoxybenzène était facilitée par l'usage intermédiaire de dérivés trialkylsiliciés selon la réaction

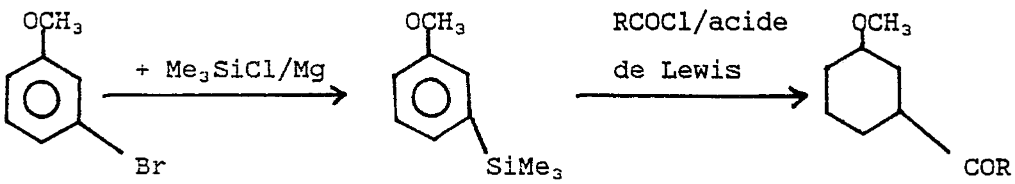


Il est apparu, lors de la reproduction de ce type de réaction, que l'on n'obtenait pas de dérivé méta-substitué, mais en majorité un dérivé parasubstitué, substitution classique sur des dérivés activés par un groupe alcoxyle. Il a même été isolé des dérivés porteurs à la fois du motif triméthylsilyle et du groupement acyle, ce qui prouve l'absence, dans ce cas, de l'effet d'orientation du groupe triméthylsilyle sur l'acylation, appelée de manière plus commune "l'effet ipso".

Par ailleurs, il avait été montré, dans le cas du toluène, que l'effet ipso pouvait s'exercer sur la position méta. Mais pour accéder au méta-triméthylsilyltoluène il fallait silyler le méta-bromotoluène et cette observation suppose donc que, pour fonctionnaliser en méta le toluène, il fallait donc partir d'un dérivé préalablement fonctionnalisé en méta, ce qui, bien évidemment diminue considérablement l'intérêt d'une telle démarche au point de vue synthétique.

Il a été découvert, et cela pour la première fois, que l'on pouvait fonctionnaliser directement en méta des alkylbenzènes ou le fluorobenzène en mettant en jeu le processus réactionnel suivant :

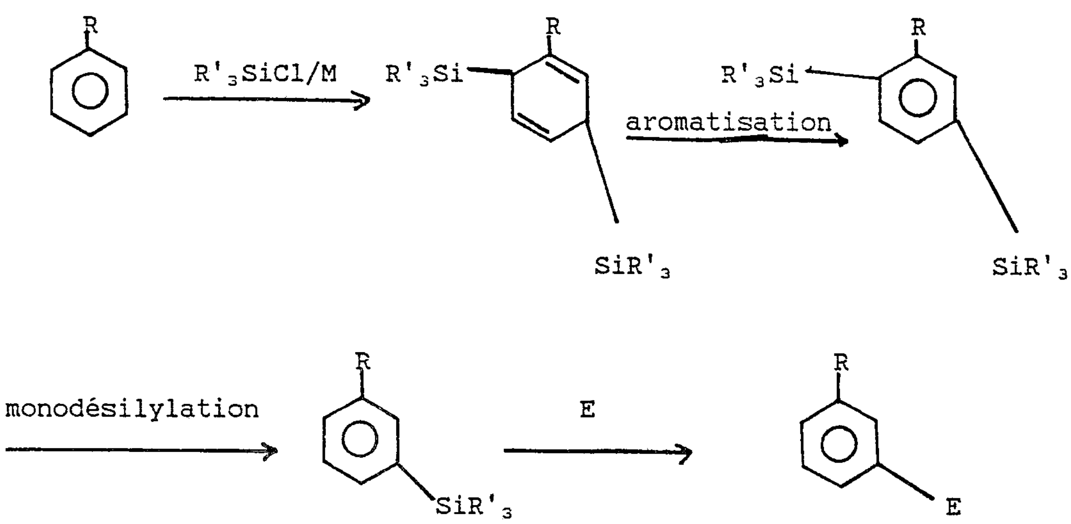


avec R = alkyle ou fluor E = agent électrophile X = halogène R'= méthyle ou éthyle M = Na, Li ou Mg

Le procédé de la présente invention consiste à partir de fluoro- ou d'alkylbenzènes :

- dans une première étape à mettre en contact, cette matière première avec un halogénure de trialkylsilane en présence d'un métal alcalin ou de magnésium (M),
- dans une deuxième étape, à aromatiser le dérivé obtenu à la première étape,
- dans une troisième étape, on effectue une monodésilylation sélective en ortho du groupe alkyle ou fluoro-,
- dans une quatrième étape, on substitue le groupement silyl du dérivé obtenu à la troisième étape par un agent électrophile.

La première étape du procédé est réalisée par condensation sur un fluoro- ou un alkylbenzène d'un halogénotrialkylsilane, de préférence un chlorotrialkylsilane, dans un solvant éthéré, tel que notamment le tétrahydrofuranne, en présence de magnésium ou d'un métal alcalin, par exemple le lithium ou le sodium à basse température. On obtient l'alkyl-1 ou le fluoro-1 bis(trialkylsilyl)-3,6 cyclohexadiène -1,4 selon la réaction :

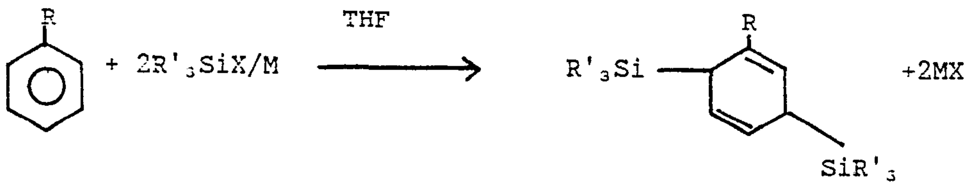


avec R = alkyle ou fluor,
R' = méthyle ou éthyle,
X = chlore,
M = Na, Li ou Mg

On utilise de préférence, au cours de cette étape, le lithium finement granulé dans du tétrahydrofuranne anhydre, maintenu à une température de préférence comprise entre 0 et 5°C.

La deuxième étape du procédé, qui consiste à aromatiser le cyclohexadiène obtenu à la première étape, est réalisée au moyen d'un agent oxydant qui peut être le soufre ou l'air, ceci n'étant pas limitatif.

La troisième étape du procédé, objet de la protection demandée, consiste à réaliser une monodésilylation sélective en ortho- du dérivé obtenu à la deuxième étape.

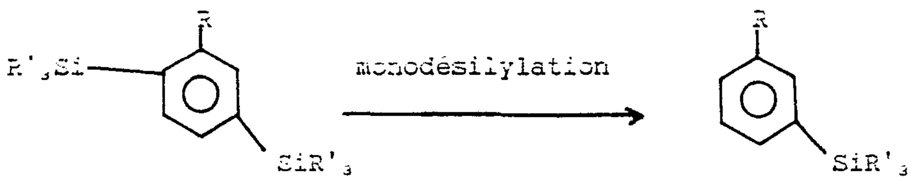


1) Selon un premier procédé de désilylation, cette étape est réalisée en présence d'une base. Cette base est choisie notamment parmi les fluorures alcalins ou alcalinoterreux, les alcoolates, les hydroxydes alcalins ou d'ammonium, les ammoniums quaternaires.

On peut citer parmi les fluorures alcalins :
- le fluorure de sodium,
- le fluorure de potassium,
- le fluorure de césium.

On peut aussi citer :
- le difluorure de calcium,
- les fluorures d'ammonium quaternaire
- les bifluorures alcalins,
- les fluorures de phosphonium ...

On peut citer parmi les alcoolates :
- l'éthylate de sodium,
- le tertiobutylate de potassium.

2) Selon un deuxième procédé de désilylation, on peut utiliser comme agent les acides forts tels que l'acide trifluoroacétique, l'acide fluorosulfonique, l'acide trifluorométhane sulfonique.

On préfère, lorsque R représente le fluor, utiliser le premier cas de désilylation et lorsque R représente un groupe alkyle, le deuxième procédé de désilylation.

Le solvant de l'étape de désilylation est choisi lors de l'utilisation de bases comme agent de désilylation (point 1, ci-dessus) parmi les solvants aprotiques polaires. On peut citer parmi ceux-ci :
- le diméthylacétamide (DMA),
- le diméthylformamide (DMF),
- la N- méthylpyrrolidone-2 (NMP),
- le diméthylsulfoxyde (DMSO).

Du point de vue conditions réactionnelles, il est particulièrement avantageux de réaliser cette réaction à une température comprise entre la température ambiante et le reflux du solvant.

Lors d'une désilylation dans les conditions indiquées au point 2, le solvant est choisi de préférence parmi les solvants aromatiques et aliphatiques éventuellement halogénes.

La quatrième étape du procédé consiste à fonctionnaliser le produit monosilicié obtenu à la troisième étape. Cette fonctionnalisation se fait par échange entre un agent électrophile et le groupe $-SiR'_3$. Cet échange peut

se faire avec l'ensemble des agents électrophiles tels que par exemple :
les agents d'acylation et notamment :
- les halogénures d'acides carboxyliques ou sulfoniques,
- les anhydrides d'acides carboxyliques ou sulfoniques,
ou d'autres agents tels que
- le brome,
- ICl,
- l'hypochlorite de sodium,
- le chlorosulfonate de triméthylsilyle,
- le chlorure de sulfuryle,
- le chlorure de sulfamoyle,
- les tétrafluoroborates de nitronium et de nitrosonium,
- le chlorure de tertiobutyle,
- les sels d'iminium.

En ce qui concerne la fonctionnalisation par acylation, il est parfois préférable d'opérer en présence d'un acide de Lewis.

L'acide de Lewis est choisi notamment parmi le chlorure d'aluminium, le trifluorure de bore, le tétrachlorure de titane et le tétrachlorure d'étain.

La fonctionnalisation du dérivé monosilicié est réalisée dans la majorité des solvants organiques à l'exclusion des solvants basiques. On peut citer à titre d'exemples les alcanes, le disulfure de carbone, les nitroalcanes, les nitriles, les solvants haloaromatiques, nitroaromatiques, ainsi que tous les solvants chloroaliphatiques.

Le produit obtenu est extrait du milieu réactionnel par toute technique connue de l'homme de l'art et notamment par extraction à l'aide de solvants.

Parmi les produits obtenus dans le cadre de la présente invention, on peut citer notamment :
- l'iodo-3 éthylbenzène,
- l'éthyl-3 benzènesulfonate de sodium,
- l'acétyl-3 éthylbenzène,
- le benzoyl-3 éthylbenzène,
- le sénécioyl-3 éthylbenzène,
- l'acétyl-3 fluorobenzène,
- le sénecioyl-3 fluorobenzène,
- le benzoyl-3 fluorobenzène,
- l'iodo-3 fluorobenzène,
- le fluoro-3 benzène sulfonate de sodium.

La présente invention va être plus précisément décrite à l'aide des exemples suivants qui ne doivent en aucun cas être considérés comme limitatifs de l'invention.

EXEMPLE 1 - FONCTIONNALISATION EN META DU FLUOROBENZENE

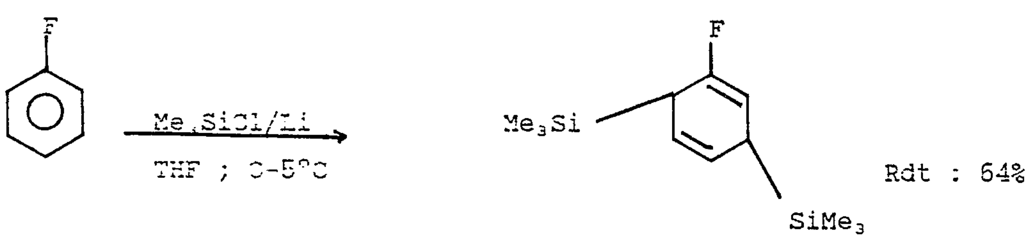


Appareillage :

Il est constitué d'un ballon de Grignard de 500 ml à 3 tubulures, muni d'une agitation magnétique, d'une gaine thermométrique, d'une ampoule à brome isobare et d'un réfrigérant ascendant relié à une colonne à chlorure de calcium. Le réacteur est équipé de façon à travailler sous balayage d'argon.

Mode opératoire :

A une suspension de 3,81 g (0,55 mol) de lithium, finement granulé, dans 225 ml de THF, déperoxydé anhydre, et 60 g de $Me_3SiCl$, fraîchement distillé, on ajoute, goutte à goutte, avec agitation, 14,4 g (0,15 mol) de fluorobenzène en refroidissant de telle manière que le milieu réactionnel soit maintenu entre 0 et 5°C. L'addition terminée, l'agitation est poursuivie 24 heures en maintenant la température vers 5-10°C. Le réaction terminée, le Li restant et LiCl sont filtrés et le filtrat évaporé sous le vide de la trompe à eau à 30°C. Dans le cas (le plus fréquent) où LiCl précipite à nouveau, on ajoute 150 ml de pentane sec qui achève la précipitation de LiCl. Après filtration et évaporation du filtrat, on recueille par distillation le produit attendu ($Eb_{0,5}$

$_{mm}$ = 76-78°C ; F = 42-44°C), avec un rendement de 64 %.

1.2. - AROMATISATION DU DERIVE CYCLOHEXADIENIQUE

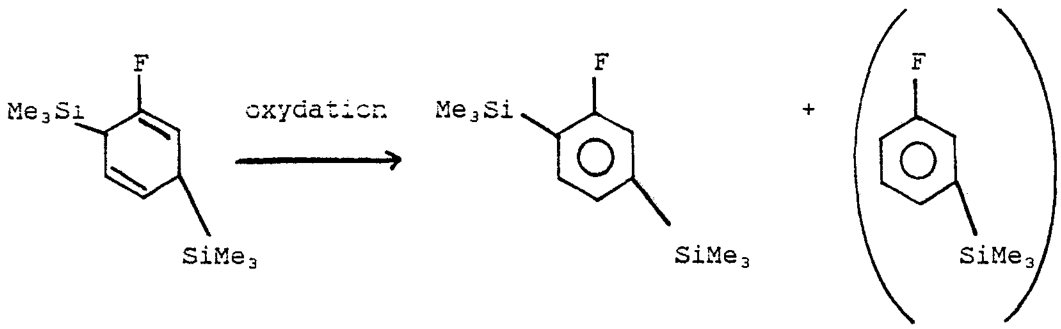


1.2.1 - Aromatisation au soufre

Appareillage :
Ballon de 100 ml muni d'un réfrigérant à reflux et d'une agitation magnétique.

Mode opératoire :
On mélange 15,0 g (62 mmol) de diène avec 7,0 g (220 mmol) de soufre dans 45 ml de toluène. On chauffe au reflux du toluène pendant 18 heures. On évapore le toluène, puis on reprend avec 100 ml de pentane et on filtre afin d'éliminer le soufre en excès. Après évaporation du solvant, le résidu est recristallisé dans l'éthanol. On obtient 7,9 g de cristaux (rendement 53 %). $T_F$ = 91°C (EtOH).

1.2.2 - Aromatisation à l'air

Appareillage :
Erlenmeyer de 1 l irradié par une lampe visible de 150 W, refroidi par un ventilateur.

Mode opératoire :
Dans un Erlenmeyer de 1 l, on introduit 20,0 g (82 mmol) de diène ; après l'avoir bouché, on l'irradie pendant 11 jours en éliminant régulièrement l'eau oxygénée formée. On recristallise le bis(triméthylsilyl)-2,5 fluorobenzène dans l'éthanol ; 14,2 g (72%).

1.3 - MONODESILYLATION DU FLUOROBENZENE DISILYLE

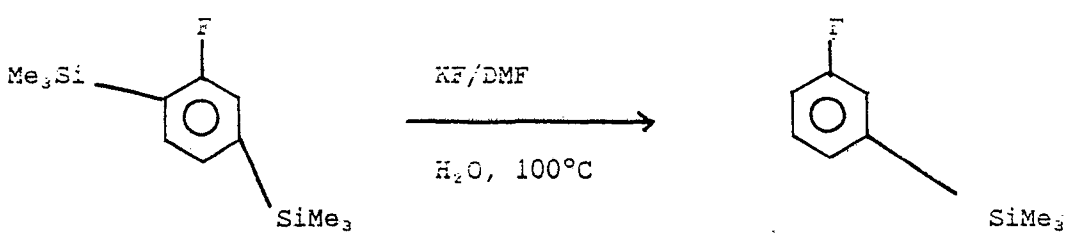


Appareillage :
Ballon de 500 ml muni d'un réfrigérant à reflux.

Mode opératoire:
A 20,0 g (83 mmol) de m-triméthylsilylfluorobenzène en solution dans 250 ml de DMF sont ajoutés 7,2 g (125 mmol) de fluorure de potassium et 1,5 g (83 mmol) d'eau. Le mélange est chauffé à 100°C pendant 17 heures ; après refroidissement, il est versé dans 300 ml d'eau. La phase aqueuse est extraite avec 100 ml de pentane. Les phases organiques sont rassemblées et lavées avec 150 ml d'eau puis séchées sur $Na_2SO_4$. Après vaporation des solvants, on recueille 12,6 g (rendement 91 %) du produit attendu.

1.4 - FONCTIONNALISATION DU BIS(TRIMETHYLSILYL)-2,5 FLUOROBENZENE

1.4.1 - Réactions d'acylation

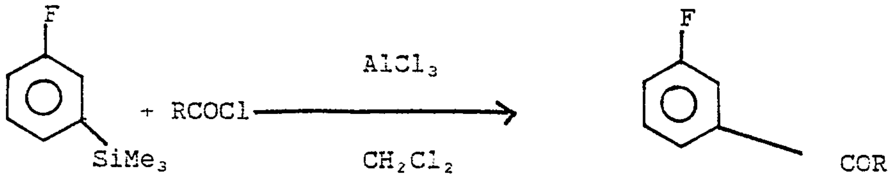


Appareillage :

Ballon de 100 ml, à tubulure latérale, sous balayage d'argon, muni d'une ampoule isobare et d'une agitation magnétique.

Mode opératoire :

A 1,6 g (12 mmol) de chlorure d'aluminium en solution dans 10 ml de $CH_2Cl_2$ on ajoute, à 0° C, 1,2 mmol de chlorure d'acide . On poursuit l'agitation à 0° C pendant 15 mn. Le milieu étant à la température convenable, 2 g (12 mmol) de triméthylsilyl-3 fluorobenzène en solution dans 10 ml de $CH_2Cl_2$ sont ajoutés. On laisse sous agitation à la température et pendant la durée indiquée. Le mélange est hydrolysé en le versant dans 50 ml d'une solution glacée de $NH_4Cl$ à 10 %. La phase organique est séparée et la phase aqueuse lavée avec 30 ml de pentane. Les phases organiques sont rassemblées et lavées jusqu'à neutralité avec une solution de $NaHCO_3$, puis séchées sur $Na_2SO_4$. Les solvants sont évaporés.

| Chlorure d'acide | T° (C°) | Durée (h) |
|---|---|---|
| Chlorure d'acétyle | 0 | 5 |
| Chlorure de sénécioyle | reflux | 16 |
| Chlorure de benzoyle | reflux | 48 |

Les produits formés lors de la benzoylation ont été séparés par chromatographie sur colonne de silice (70-230 Mesh), (30 g de silice pour 1 g de produit) en éluant avec un mélange toluène/pentane (1/4)

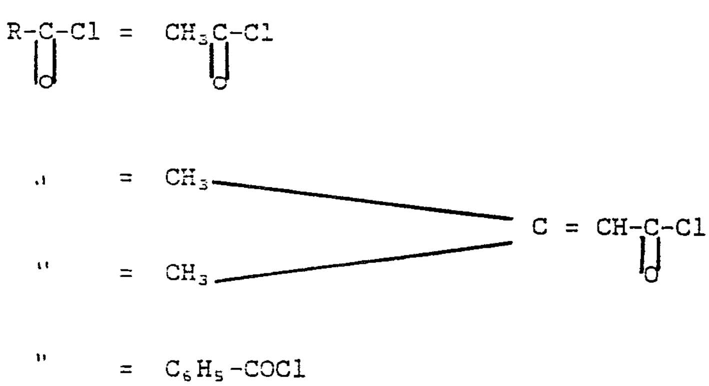

Rdt = 80 %

Rdt = 54 %

Rdt = 41 %

1.4.2 - Ioduration

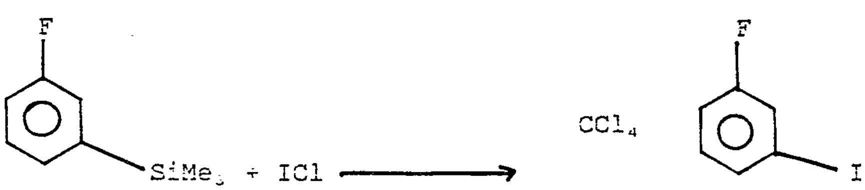

Appareillage :

Ballon de 100 ml à tubulure latérale sous balayage d'argon muni d'une ampoule isobare.

Mode opératoire :

On ajoute 1,5 g (9 mmole) de monochlorure d'iode en solution dans 10 ml de $CCl_4$ à 1,5 g (9 mmole) de triméthylsilyl-3 fluorobenzène en solution dans 40 ml de $CCl_4$, à température ambiante. Le mélange est porté au reflux pendant 48 heures, puis refroidi et versé dans 100 $cm^3$ d'eau, extraction à l'éther ; la phase organique est lavée avec une solution de $Na_2S_2O_3$. On sèche sur $Na_2SO_4$, puis on évapore les solvants. On recueille 1,1 g de produit (rendement 83 %).

1.4.3 - Bromation

Appareillage :

Un ballon de 50 ml muni d'une agitation magnétique et d'un réfrigérant ascendant.

Mode opératoire :

0,16 g (0,001 mol) de brome sont additionnés à 0,17 g (0,001 mol) de triméthylsilyle-3 fluorobenzène en solution dans 5 ml de dibromométhane à température ambiante. Le mélange réactionnel est ensuite chauffé à 80° C pendant 3 heures ; puis il est hydrolysé avec 10 ml d'eau et extrait avec 3 x 5 ml d'éther di isopropylique.

Le taux de transformation du triméthylsilyle-3 fluorobenzène est de 34 %.

Le rendement sur produit transformé en méta bromofluorobenzène est de 47 %.

1.4.4 - Sulfonation

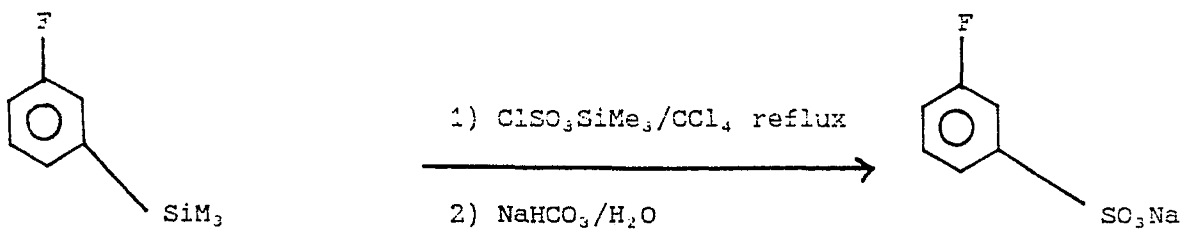


Appareillage

Un ballon de 100 ml de tubulure latérale muni d'une ampoule isobare et d'une agitation magnétique.

Mode opératoire :

Une solution de 1,7 g (9 mmol) de chlorosulfonate de triméthylsilyle dans 40 ml de $CCl_4$ est ajoutée à une solution de 1,5 g (9 mmol) de triméthylsilyl-3 fluorobenzène dans 140 ml de $CCl_4$ à température ambiante. Le mélange est chauffé au reflux du $CCl_4$ pendant 16 heures, puis hydrolysé avec 50 ml d'une solution de $NaHCO_3$. La solution aqueuse est lavée à l'éther puis évaporée. Le sel de sodium de l'acide sulfonique est recristallisé dans l'éthanol après filtration à chaud pour éliminer les sels minéraux. On recueille 1,3 g de produit (rendement 76 %).

1.4.5. -Sulfamoylation

Dans le même appareillage que précédemment 1,2 g (10 mmol) de chlorure de sulfamoyle en solution dans 5 ml de $CH_2Cl_2$ sont ajoutés à une solution de 1,3 g (10 mmol) de chlorure d'aluminium dans 5 ml de chlorure de méthylène à 0° C. L'agitation est poursuivie pendant 30 mn. 1,7 g (10 mmol) de trimétylsilyl-3 fluorobenzène dans 10 ml de chlorure de méthylène sont ajoutés à -40° C. Après addition, le milieu est progressivement ramené à température ambiante et maintenu 15 heures. Après hydrolyse, extraction et évaporation des solvants, le produit attendu est recristallisé dans le toluène avec un rendement de 57 %.

1.4.6. - Tertiobutylation

Appareillage :

Tube SCHOTT

Mode opératoire :

A température ambiante sont mélangés 0,0990 g (0,001 mol) de t-BuCl à 0,1349 g (0,001 mol) de $AlCl_3$ en solution dans 2 ml de nitrobenzène, puis 0,1726 g (0,001 mol) de triméthylsilyl-3 fluorobenzène. Le tube est

ensuite chauffé à 50°C pendant 5 heures. Après hydrolyse (10 ml d'eau) et extraction avec l'éther diisopropylique (3 x 5 ml), le mélange est ramené à un volume connu (25 ml).

On obtient 29 % de méta tertiobutylfluorobenzène et différents composés provenant de la déméthylation du dérivé précédent.

EXEMPLE 2

On reproduit l'exemple 1 en remplaçant le fluorobenzène par l'éthylbenzène.

2.1 - La double silylation en ortho et en méta donne un rendement en bis(triméthylsilyl)-3,6 éthylcyclohexadiène-1,4 de 75 %.

2.2.1 - L'aromatisation est effectuée à l'air comme dans la partie 1.2.2. de l'exemple 1 pendant 96 heures et fournit un rendement en bis(triméthylsilyl)-2,5 éthylbenzène de 64 %.

2.2.2 - L'aromatisation effectuée dans le tétrachlorure de carbone en présence de rayonnements UV pendant 40 heures donne un rendement en bis(triméthylsilyl)-2,5 éthylbenzène de 63 %.

2.2.3 - Par action du p-chloranile : une solution de 5 g (20 mmol) de dérivé cyclohexadiénique et de 10,3 g de p-chloranile dans 200 ml de toluène est chauffée au reflux pendant 40 heures. Après évaporation du toluène, le résidu est filtré sur un verre fritté rempli de silice et élué avec 150 ml de pentane. Après élimination du solvant de distillation, nous avons obtenu le produit avec 33 % de rendement.

2.3 - La monodésilylation de l'éthylbenzène disilicié est effectuée dans un mélange d'acide trifluoracétique et de tétrachlorure de carbone au reflux.

Appareillage :

Ballon de 50 ml équipé d'un réfrigérant ascendant.

Mode opératoire :

A une solution de 2,0 g (8 mmol) de dérivé disilicié dans 15 ml de $CCl_4$ sont ajoutés, à température ambiante, 0,9 g (8 mmol) d'acide trifluoroacétique. Le mélange est chauffé au reflux pendant 15 heures. Après extraction au pentane, lavage de la phase organique jusqu'à neutralisation, puis séchage et évaporation du solvant, l'éthylbenzène métasilicié est obtenu avec un rendement quantitatif.

2.4 - La fonctionnalisation du triméthylsilyl-3 éthylbenzène est effectuée conformément au tableau suivant dans lequel on met en oeuvre 1 mole de monosilyléthylbenzène pour x mole d'agent de fonctionnalisation.

L'appareillage et les quantités de produits mis en oeuvre (x) sont identiques à ceux décrits dans le cas du fluorobenzène. Les conditions opératoires (température, durée et rendement) sont indiquées dans le tableau ci-après.

FONCTIONNALISATION DE 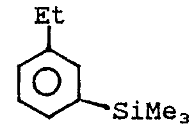

| Agent de fonctionnalisation | Solvant | Produit obtenu | Rendement |
|---|---|---|---|
| ICl | $CCL_4$ (reflux) 48 heures | 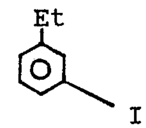 | 94 |
| 1) $ClSO_3SiMe_3$ ) ) 2) $H_2O/NaHCO_3$ ) | $CCl_4$ (reflux) 16 heures | 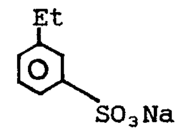 | 76 |
| $MeCOCl/AlCl_3$ | $CH_2Cl_2$ -20°C 2 heures | 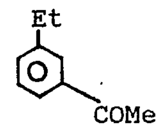 | 69 |
| $C_6H_5COCl/AlCl_3$ | $CH_2Cl_2$ (reflux) 12 heures | 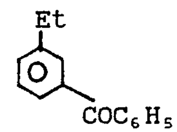 | 60 |
| 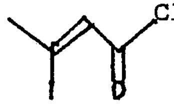 $Cl/AlCl_3$ | $CH_2Cl_2$ (20°C) 12 heures | 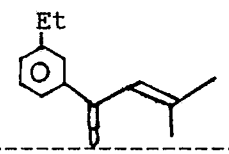 | 80 |

## Revendications

1. Procédé de de subsitution en position méta de fluoro- ou d'alkylbenzènes caractérisé en ce que dans une première étape, on met en contact dans un solvant un fluoro- ou un alkylbenzène avec un halogénure de trialkylsilane en présence d'un métal alcalin ou de magnésium
dans une deuxième étape on aromatise le dérivé obtenu à la première étape
dans une troisième étape on effectue une monodésilylation en ortho du groupe fluoro- ou alkyl
dans une quatrième étape on substitue le groupement silyle du dérivé monosilicié par un agent électrophile.

2. Procédé selon la revendication 1 caractérisé en ce que la monodésilylation des fluoro ou des alkyl benzènes-2,5-disiliciés est effectuée par un fluorure alcalin ou alcalinoterreux, un hydroxyde alcalin, d'ammonium, un alcoolate, un ammonium quaternaire, l'acide trifluoroacetique, l'acide fluorosulfonique ou l'acide trifluorométhane sulfonique.

3. Procédé selon la revendication 1 caractérisé en ce que l'agent électrophile est choisi parmi les acides, les halogénures d'acides, les anhydrides d'acides, ICl, le brome, l'hypochlorite de sodium, le chlorure de sulfuryle, le chlorosulfonate de triméthylsilyle, le chlorure de sulfamoyle, les tétrafluoroborates de nitronium, le chlorure de tertiobutyle.

4. Procédé selon la revendication 3 caractérisé en ce que les acides et leurs dérivés sont choisis parmi les acides carboxyliques, les acids sulfoniques...

5. Procédé selon la revendication 4 caractérisé en ce que la condensation est effectuée en présence d'un acide de Lewis.

6. Procédé selon la revendication 5 caractérisé en ce que l'acide de Lewis est choisi parmi le chlorure d'aluminium, le trifluorure de bore, le tétrachlorure de titane, le tétrachlorure d'étain.

7. Procédé selon la revendication 1 caractérisé en ce que la 1ère étape est effectuée dans un solvant aprotique polaire.

8. Procédé selon la revendication 1 caractérisé en ce que le métal alcalin choisi est le lithium.

9. Procédé selon les revendications 1 et 2 caractérisé en ce que la 3ème étape est effectuée dans un solvant choisi parmi le diméthylacétamide, le diméthylformamide, la N-méthylpyrrolidone-2, le diméthylsulfoxyde ; un solvant aliphatique ou aromatique éventuellement hologéné ou nitré.

Office européen des brevets

# RAPPORT DE RECHERCHE EUROPEENNE

Numero de la demande

EP 89 40 1698

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | J. CHEM. RESEARCH (S), 1980, pages 236-237; G. FELIX et al.: "Regiospecific functionalisation of toluene and m-xylene" * En entier * | 1-9 | C 07 B 37/04<br>C 07 B 39/00<br>C 07 B 45/04<br>C 07 C 25/02<br>C 07 C 49/76<br>C 07 C 309/39<br>C 07 C 311/16<br>C 07 F 7/08<br>C 07 C 17/22<br>C 07 C 45/00 |
| Y | JOURNAL OF THE CHEMICAL SOCIETY, novembre 1959, page 3640; C. EABORN et al.: "Aromatic reactivity. Part VII. Additivity of effects of methyl substituents in protodesilylation" * En entier * | 1-9 | |
| | | | **DOMAINES TECHNIQUES RECHERCHES (Int. Cl.5)** |
| | | | C 07 B<br>C 07 F |

Le présent rapport a été établi pour toutes les revendications

| Lieu de la recherche | Date d'achèvement de la recherche | Examinateur |
|---|---|---|
| LA HAYE | 25-10-1989 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant